# NOUVEAU FASCICULE DE BREVET EUROPEEN

(11) **EP 0 523 120 B2**
(45) Date de publication et mention de la décision concernant l'opposition: **02.11.2000**
(45) Mention de la délivrance du brevet: 01.06.1994
(21) Numéro de dépôt: 91907048.2
(22) Date de dépôt: 22.03.1991
(51) Int. Cl.: C08J 3/12, C09D 101/28, A61K 9/36, A23P 1/08

(54) **PRODUIT FILMOGENE DESTINE A L'ENROBAGE DES FORMES SOLIDES**
Filmformendes Produkt zur Beschichtung von Feststoffen
FILM-FORMING PRODUCT INTENDED FOR COATING SOLID FORMS

(30) Priorité: 27.03.1990 FR 9003915
(43) Date de publication de la demande: 20.01.1993
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: TROUVE, Gérard, F-81100 Castres (FR)
(74) Mandataire: Hubert, Philippe
(86) Numéro de dépôt international: FR9100232
(87) Numéro de publication internationale: WO9114729

(56) Documents cités:
- EP-A- 0 178 138
- EP-A- 0 318 314
- WO-A-85/01207
- FR-A- 2 548 675
- GB-A- 2 065 691
- US-A- 4 513 019
- US-A- 4 576 646
- US-A- 4 665 648
- Banque de donnees WPIL, abstract no. 86-090761, Derwent Publications Ltd. London, GB ; & JP-A-61036206 (KANEBO KK.) 20.02.1986
- Pharmacopée européenne, "Granulata", p. 499, 1990 et "pulveres", p. 527, 1986
- M. Aulton "Pharmaceutics: The Science of Dosage Form Design", 1988, pp. 308-309
- L. lachman et al. "The Theory and Practice of Industrial Pharmacy", 1986, pp. 76, 77, 321, 327, 328

## Description

La présente invention concerne généralement un nouveau produit filmogène, destiné à l'enrobage des formes solides de préférence de produits agricoles, pharmaceutiques ou alimentaires, son procédé de préparation et les produits revêtus à l'aide de ce produit.

On connaît de nombreux produits filmogènes destinés à l'enrobage des formes solides de produits pharmaceutiques ou alimentaires. Ces produits se différencient essentiellement par leur composition ainsi que par la forme sous laquelle ils se présentent.

D'une façon générale, ces produits contiennent des polymères cellulosiques, des pigments de coloration, des plastifiants et éventuellement des charges diverses.

Le brevet français no. 2.470.598 décrit un produit comportant ces différents ingrédients, et qui se présente sous la forme d'un mélange sec exempt de solvant. Ce produit peut être facilement dispersé dans des solvants organiques ou aqueux pour réaliser une solution filmogène susceptible d'être pulvérisée sur les produits à revêtir, mais présente l'inconvénient de générer des poussières colorées salissantes lors de sa manipulation ou de son transfert.

En outre, ce produit s'écoule difficilement et en raison des différences importantes de morphologie et de granulométrie de ses divers constituants, on observe des variations de couleurs indésirables du revêtement.

Le brevet américain no. 4.816.298 décrit un procédé pour le pelliculage de comprimés comprenant la préparation de particules constituées de polymère thermofusible, de plastifiant et de pigment de coloration.

Ces particules de diamètre compris entre environ 1 et environ 0,1 mm ne provoquent pas de poussière, sont stables et se dispersent facilement dans l'eau froide.

Cependant, ce procédé nécessite l'utilisation de quantités très importantes (supérieures à 30% en poids) de plastifiant pour "solubiliser" le polymère et obtenir une température de fusion-extrusion qui ne soit pas excessive. En outre, dans les exemples indiqués dans ce document, l'extrusion est réalisée à une température de l'ordre de 90°C, qui peut être dommageable pour les colorants ou laques nécessaires à la coloration des films.

Le brevet français 2.548.675 de la Demanderesse décrit un produit filmogène contenant de l'α-cellulose et se présentant sous la forme d'un granulé non pulvérulent d'un diamètre d'environ 0,5 à 1 mm, et dont les différents constituants sont répartis de façon homogène.

Le produit ainsi obtenu peut être très facilement dispersé et n'entraîne aucun risque de stratification.

Par ailleurs, à viscosité égale, les solutions d'enrobage obtenues à partir de ce produit sont plus concentrées que celles réalisées à partir des produits décrits dans les documents FR 2.470.598 et US 4.816.298, et permettent d'enrober plus rapidement les produits à revêtir.

Cependant, les films obtenus à partir d'un tel produit ont un aspect mat et il est assez difficile de les rendre brillants.

Il est important de noter que l'α-cellulose est considérée et présentée comme un constituant essentiel pour la réalisation de granulés, en raison du rôle de liant joué par celle-ci et parce que l'α-cellulose assure également une bonne adhérence du film d'enrobage sur le produit à revêtir.

La présente invention est basée sur la constatation que la présence d'α-cellulose dans les granulés, réalisés conformément au brevet FR 2.548.675, conduit à un film présentant une surface relativement irrégulière ne permettant pas de conférer un aspect brillant audit film.

L'invention est ensuite fondée sur la découverte du fait qu'il est possible de réaliser un produit filmogène, exempt d'α-cellulose, sous la forme de particules granulées, permettant l'obtention de films réguliers et non mats.

La présente invention a donc pour but de résoudre le problème technique consistant en la fourniture d'un nouveau produit filmogène constitué de particules granulées susceptibles de se disperser facilement dans un solvant aqueux ou organique et permettant l'obtention d'un film régulier pouvant être rendu brillant.

La solution, conforme à la présente invention, pour résoudre ce problème technique, consiste en un produit filmogène exempt d'α-cellulose, destiné à l'enrobage des formes solides, caractérisé en ce qu'il se présente sous forme de particules granulées homogènes, susceptibles de se disperser facilement dans un solvant aqueux ou organique et permettant l'obtention d'un film régulier et non mat, et en ce que sa matière sèche comprend :
- au moins une substance filmogène non toxique ingérable en une quantité en poids comprise entre 30 et 95 % ;
- au moins un pigment de coloration, en une quantité comprise entre 5 et 50 % en poids ; et/ou
- au moins un agent plastifiant alimentaire, en une quantité inférieure ou égale à 25 % en poids, et en ce qu'il est obtenu par un procédé comprenant le mouillage d'au moins une majeure partie, supérieure ou égale à environ 95 % en poids du poids total, de la substance filmogène sous forme de poudre, au moyen d'une solution de granulation liante, dans un mélangeur-granulateur ou dans un lit d'air fluidisé.

Avantageusement, la matière sèche précitée comprend :
- au moins une substance filmogène non toxique ingérable, en un quantité en poids comprise entre 60 et 80 %, de préférence entre 65 et 75 % ;
- au moins un pigment de coloration, en une quantité en poids comprise entre 10 et 40 %, de préférence entre 15 et 35 % ; et/ou
- au moins un agent plastifiant alimentaire, en une quantité en poids inférieure ou égale à 25 %, de préférence comprise entre environ 5 et 15 % et encore de préférence de 10 %.

Selon une caractéristique particulière, le produit filmogène conforme à l'invention comprend en outre au moins une charge choisie parmi un agent opacifiant, un agent hydrofugeant ou encore un agent mouillant.

La substance filmogène utilisée dans le produit conforme à l'invention peut être constituée par un ou plusieurs agents filmogènes ingérables connus et précédemment décrits, par exemple dans les brevets mentionnés ci-dessus.

Parmi ces produits, on mentionnera plus spécialement comme dérivés cellulosiques utilisables, les alkyl éthers ou alkyl esters de cellulose comme par exemple, méthylcellulose, hydroxypropylcellulose, hydroxypropylméthylcellulose, acétophtalate de cellulose, acétate de cellulose ou éthylcellulose ; la polyvinylpyrrolidone ; la zéine ; les polymères acryliques.

Avantageusement, on utilisera une hydroxypropylméthylcellulose, de viscosité comprise entre 3 et 15 cP à température ambiante, en solution à 2 % en poids dans l'eau.

Les pigments de coloration susceptibles d'être utilisés conformément à l'invention peuvent être choisis parmi les pigments utilisés jusqu'alors dans la fabrication de produits filmogènes destinés au revêtement de forme solide de produits pharmaceutiques ou alimentaires.

Des exemples de ces pigments sont décrits en particulier dans les brevets mentionnés précédemment.

Parmi ces pigments, on utilisera tous pigments ou colorants (solubles ou sous forme de laques) de qualité alimentaire et notamment le dioxyde de titane.

Les agents plastifiants, susceptibles de constituer en partie le produit filmogène conforme à l'invention, peuvent être choisis parmi les agents plastifiants connus et décrits notamment dans les brevets mentionnés précédemment. A titre d'exemple d'agent plastifiant pouvant être utilisé, on peut citer le glycérol, le polyéthylèneglycol de poids moléculaire compris entre 400 et 10000, la glycérine, le propylèneglycol, les sucres notamment le glucose, les maltodextrines, les monoglycérides acétylés, les esters d'acide citrique ou lactique, les acides gras éthoxylés.

Le produit filmogène conforme à l'invention peut comporter en outre au moins une charge habituellement utilisée pour modifier les propriétés du matériau d'enrobage et la protection dudit matériau. A titre d'exemple de charge, on mentionnera les agents opacifiants comme par exemple le talc, hydrofugeants comme par exemple les acides gras et leurs dérivés, les polymères silicone et les agents mouillants comme par exemple les tensio-actifs alimentaires traditionnels.

Selon un second aspect, la présente invention a pour objet un procédé pour la préparation d'un produit filmogène sous forme de particules granulées tel que précédemment défini caractérisé en ce qu'il comprend le mouillage d'au moins une majeure partie, supérieure à environ 95 % en poids du poids total, de la substance filmogène sous forme de poudre, au moyen d'une solution de granulation liante dans un dispositif mélangeur-granulateur ou dans un lit d'air fluidisé.

Lorsque le mouillage est réalisé dans un lit d'air fluidisé, les particules granulées sont obtenues directement.

A titre d'exemple de dispositif à lit d'air fluidisé pouvant être utilisé, on peut citer les dispositifs connus sous la dénomination Glatt ou Aeromatic.

Lorsque le mouillage précité est réalisé dans un mélangeur-granulateur, la masse humide obtenue est subséquemment broyée, puis séchée et éventuellement tamisée afin d'obtenir des particules granulées homogènes. A titre d'exemple de dispositif mélangeur-granulateur pouvant être utilisé, on peut citer les dispositifs connus sous la dénomination Diosna ou Lodige.

Selon une caractéristique particulière du procédé conforme à l'invention, la solution de granulation liante précitée comprend, dans un solvant, tel que de préférence l'eau :
- la totalité des pigments de coloration ;
- une faible partie, comprise entre environ 0,01 et environ 5 % en poids du poids total, de la substance filmogène ;
- et éventuellement l'agent plastifiant.

La dispersion d'une faible partie d'agent filmogène dans le liant de granulation permet d'obtenir une solution liante plus homogène et plus stable dans le temps, ne nécessitant pas d'être agitée lors de l'opération de granulation. Cette dispersion permet en outre d'augmenter le pouvoir liant de la solution par rapport à une simple dispersion de pigment dans le solvant. En effet, on a observé que le filmogène parfaitement hydraté, peut mouiller et enrober les particules de pigment et favoriser ainsi la formation de granulés homogènes lors du mélange avec les poudres.

Ainsi, la dispersion d'une faible partie d'agent filmogène dans le liant de granulation permet de préparer des granulés exempts d'α-cellulose.

Selon une autre caractéristique particulière du procédé conforme à l'invention , la solution de granulation liante précitée est obtenue par mélange d'une dispersion "mère" contenant une partie, comprise entre environ 10 et environ 50 % du poids total, du solvant ; les pigments de coloration et la faible partie précitée d'agent filmogène et d'une dispersion contenant la partie restante du solvant et éventuellement l'agent plastifiant.

Avantageusement, préalablement au mélange de ces deux dispersions, la dispersion "mère" est broyée, de préférence dans un broyeur à billes, de façon à obtenir une granulométrie fine et homogène, par exemple inférieure à 150 µm.

Selon un troisième aspect, l'invention a pour objet des produits enrobés, caractérisés en ce que l'enrobage a été réalisé à l'aide d'un produit filmogène tel que défini précédemment. De préférence, il s'agit de produits enrobés à usage agricole, alimentaire ou pharmaceutique.

Cet enrobage peut être réalisé en dispersant le produit filmogène dans un solvant organique ou aqueux approprié pour réaliser une solution filmogène et en pulvérisant cette solution sur les produits à enrober de façon connue en soi.

L'invention sera mieux comprise à l'aide des exemples illustratifs suivants, qui sont donnés à titre non limitatifs.

### Exemple 1

On a réalisé un produit filmogène conforme à l'invention sous forme de particules granulées sans plastifiant, de la façon suivante.

On disperse 3,5 kg de dioxyde de titane de qualité alimentaire (anathase) dans 4,6 kg d'une solution aqueuse contenant 0,13 kg d'hydroxypropylméthylcellulose (6 cPs).

Cette dispersion est broyée de façon à obtenir un lait homogène exempt d'agglomérat solide.

Ce lait est introduit progressivement dans un mélangeur-granulateur Diosna V 100 contenant 6,4 kg d'hydroxypropylméthylcellulose (6 cPs).

La vitesse du mélangeur est maintenue en position 1 pendant 3 min.

0,2 kg d'eau sont alors ajoutés pour obtenir un grain plus homogène, la vitesse du mélangeur étant maintenue en position 2 pendant 1 min supplémentaire.

On introduit alors le grain humide ainsi obtenu dans un broyeur de type Tornado muni d'une grille de 5 mm et tournant à vitesse minimale. Le produit ainsi obtenu est ensuite séché dans une étuve ventilée à 110°C pendant 22 h.

On obtient ainsi une poudre granuleuse blanche comportant environ 1 % d'eau.

Les caractéristiques du produit obtenu ont été regroupées au tableau I.

Les mesures et analyses correspondantes ont été réalisées de la façon suivante :
- temps d'écoulement
   II correspond au temps nécessaire pour que 100 g de granulés placés dans un entonnoir normalisé (NF 35 032) s'écoulent totalement sans l'aide de vibration ou de choc.
- temps de mise en solution
   Dans un bêcher de 1 l, 220 mm de diamètre, on prépare une dispersion de 75 g de granulés filmogènes dans 425 g d'eau désionisée à 20°C, par agitation au moyen d'une turbine défloculeuse Raynerie d'un diamètre de 65 mm tournant à 1 500 tr/min.
   Le temps de mise en solution est le temps nécessaire pour que toute particule ou grain visible à l'oeil nu ait disparu.
- viscosité
   La dispersion préparée précédemment est maintenue sous agitation pendant 1 h.
   Puis cette dispersion est laissée au repos pendant 1 h et sa viscosité à 20°C est mesurée au moyen d'un viscosimètre Brookfield (modèle LVT) muni d'un mobile n° 2 tournant à la vitesse de 60 tr/min.
- stabilité
   Dans une centrifugeuse tournant à 3 000 tr/min et provoquant une accélération de 1 500 g, on place environ 10 ml de la solution précédemment décrite, dont on a mesuré la viscosité.
   On note le volume de culot de centrifugation V_{c} et celui du surnageant Vₛ (exprimé en pourcentage) au bout de 15 min de centrifugation.

### Préparation de comprimés enrobés

On place 500 g de comprimés dans une turbine d'enrobage Erweka tournant à 18 tr/min.

Une solution à 15 % du granulé est préparée comme indiqué précédemment et pulvérisée au moyen d'un pistolet Binks à raison de 10 g/min.

De l'air chaud à 40°C environ est insufflé dans la turbine.

Après avoir déposé environ 100 g de solution, on retire les comprimés de la turbine. Ces derniers sont parfaitement couverts d'un film blanc régulier, sans aspérités, et d'aspects brillants.

A titre de comparaison, on a mesuré les caractéristiques de deux produits filmogènes commercialisés.

Le produit Opadry YS 7802 de la société Colorcon est un mélange sec exempt de solvant selon le brevet français 2.470.598.

Le produit Sépifilm 752 de la demanderesse est une poudre granuleuse préparée selon le brevet français 2.548.695.

Les résultats sont indiqués au tableau I où est également mentionné l'aspect du film obtenu à l'aide de ces produits en suivant le protocole décrit ci-dessus.

**TABLEAU I**

| | Produit filmogène de l'exemple 1 | OPADRY YS 7802 | SEPIFILM 752 |
|---|---|---|---|
| Temps d'écoulement (s) | 12 | ne s'écoule pas | 11 |
| Temps de dissolution (min) | 14 | 6 (avec grumeaux) | 9 (sans grumeau) |
| Viscosité (mPa.s) solution à 15 % | 500 | 275 | 200 |
| Stabilité solution à 15 % | Vs = 6,7 % | Vs = 11 % | Vs = 19 % |
| | Vc = 0 | Vc = 0 | Vc = 20 % |
| Aspect du film | blanc, régulier, brillant | blanc, régulier, brillant | blanc, mat |

### Exemple 2

On prépare une dispersion par agitation dans 3,5 kg d'eau déminéralisée d'un mélange comportant 3,5 kg de dioxyde de titane (anatase) et 0,16 kg de HPMC (3 cPs).

Cette dispersion est finement broyée, puis versée au moyen d'une pompe péristaltique débitant 1,75 kg/mn dans un mélangeur-granulateur Diosna V100 contenant 3,9 kg d'HPMC (6 cPs) et 2,4 kg d'HPMC (3 cPs). Les conditions opératoires sont les mêmes que celles décrites à l'exemple 1.

On obtient après tamisage sur une grille 2 mm des particules granulées dont les caractéristiques ont été reportées au tableau II.

On a enrobé des comprimés à l'aide de ces particules granulées de la même façon qu'à l'exemple I et le film obtenu est régulier, blanc et brillant.

### Exemple 3

### A. Préparation d'un liant de granulation :

On disperse 0,75 kg d'oxyde de magnésium et 0,075 kg d'HPMC (3 cPs) dans 2 kg d'eau déminéralisée.

Cette dispersion "mère" est broyée.

On mélange cette dispersion mère broyée à une dispersion constituée de 1,675 kg de propylèneglycol (plastifiant) dans 0,5 kg d'eau.

### B. Préparation des particules granulées :

Le liant de granulation obtenu à l'étape A est suffisamment stable et homogène pour être introduit au moyen d'une pompe dans un mélangeur-granulateur Diosna V100 contenant 5 kg d'HPMC (3 cPs).

Les conditions de granulation sont identiques à celles décrites à l'exemple 1. Cependant, une quantité d'eau de finition est ajoutée pour obtenir une masse humide granulée d'aspect parfaitement homogène. Cette masse est séchée et broyée et conduit à un granulé présentant les caractéristiques regroupées au tableau II.

### Exemple 4

### Etape A : préparation d'une solution de granulation liante :

On prépare une dispersion "mère" contenant 2,94 kg de dioxyde de titane dans 2,5 kg d'eau déminéralisée.

Cette dispersion est broyée dans un broyeur à billes.

On réalise d'autre part une seconde dispersion de 0,20 kg d'HPMC (6 cPs) dans 0,5 kg d'eau chaude à laquelle on ajoute 0,91 kg de triacétine.

La dispersion mère est versée sous agitation dans la deuxième dispersion, pour obtenir un liant de granulation stable.

### Etape B : préparation de particules granulées

Le liant obtenu à l'étape A est introduit en 5 min au moyen d'une pompe péristaltique sur 5,95 kg d'HPMC (3 cPs) placée dans un mélangeur Diosna V100 tournant à la vitesse 1.

Une quantité d'eau de finition suffisante pour obtenir une masse homogène est ajoutée, le mélangeur tournant alors à la vitesse 2 pendant 1 min supplémentaire. Après broyage, séchage et tamisage comme indiqué à l'exemple 1, on obtient un granulé ayant les caractéristiques indiquées au tableau II et dont l'extrait sec est de 95 %.

### Exemple 5

On réalise ici un granulé filmogène coloré.

### Etape A : préparation d'un liant de granulation :

On prépare une dispersion dans 1,8 kg d'eau de :
- 1,771 kg de dioxyde de titane ;
- 0,018 kg d'oxyde de fer jaune ;
- 0,065 kg d'oxyde de fer noir ;
- 0,045 kg d'indigotine sous forme de laque d'alumine.

Cette dispersion "mère" est broyée.

On prépare une seconde dispersion constituée de 0,8 kg de polyéthylèneglycol 400, de 0,1 Kg d'HPMC (6 cPs) et de 0,9 kg d'eau déminéralisée.

La dispersion "mère" est mélangée dans cette seconde dispersion.

### Etape B : préparation de granulés

La solution obtenue à l'étape A est introduite en 10 min au moyen d'une pompe péristaltique dans un mélangeur-granulateur contenant 7,3 kg d'HPMC (6 cPs). Le mélange est maintenu pendant quelques minutes à la vitesse 1.

On introduit ensuite 0,3 kg d'eau déminéralisée pour obtenir une masse homogène et l'agitation est maintenue 6 min supplémentaires à la vitesse 1.

La masse humide ainsi obtenue est séchée, broyée et tamisée comme décrit à l'exemple 1, pour obtenir des particules granulées vertes, dont la matière sèche représente 97 % en poids, et dont les caractéristiques sont regroupées au tableau II.

Des essais complémentaires ont été effectués avec d'autres substances filmogènes, pigments et plastifiants dans des proportions variées qui ont permis de déterminer les paramètres généraux influençant la préparation de particules granulées conforme à l'invention et indiqués précédemment.

**TABLEAU II**

| | Produit filmogène de l'exemple 2 | Produit filmogène de l'exemple 3 | Produit filmogène de l'exemple 4 | Produit filmogène de l'exemple 5 |
|---|---|---|---|---|
| Temps d'écoulement (s) | 17 | 14 | 20 | |
| Temps de dissolution (min) | 17 | - | 14 | |
| Viscosité (mPa.s) solution à 15 % | 280 | 470 | 275 | |
| Stabilité solution à 15 % | Vs = 6,7 % | | Vs = 2,7 % | |
| | Vc = 0 | - | Vc = 0 | |
| Aspect du film | régulier, blanc, brillant | fin, régulier, opaque | régulier, blanc, brillant | |

## Revendications

1. Produit filmogène exempt d'α-cellulose, destiné à l'enrobage des formes solides, caractérisé en ce qu'il se présente sous forme de particules granulées homogènes, susceptibles de se disperser facilement dans un solvant aqueux ou organique et permettant l'obtention d'un film régulier et non mat, en ce que sa matière sèche comprend :
- au moins une substance filmogène non toxique ingérable, en une quantité en poids comprise entre 30 et 95 % ;
- au moins un pigment de coloration, en une quantité comprise entre 5 et 50 % en poids ; et/ou
- au moins un agent plastifiant alimentaire, en une quantité inférieure ou égale à 25 % en poids, et en ce qu'il est obtenu par un procédé comprenant le mouillage d'au moins une majeure partie, supérieure ou égale à environ 95 % en poids du poids total, de la substance filmogène sous forme de poudre, au moyen d'une solution de granulation liante, dans un mélangeur-granulateur ou dans un lit d'air fluidisé.

2. Produit filmogène selon la revendication 1, caractérisé en ce que sa matière sèche comprend :
- au moins une substance filmogène non toxique ingérable, en une quantité en poids comprise entre 60 et 80 %, de préférence entre 65 et 75 % ;
- au moins un pigment de coloration, en une quantité en poids comprise entre 10 et 40 %, de préférence entre 15 et 35 % ; et/ou
- au moins un agent plastifiant alimentaire, en une quantité en poids inférieure ou égale à 25 %, de préférence comprise entre environ 5 et 15 % et encore de préférence de 10 %.

3. Produit filmogène selon la revendication 1 ou 2, caractérisé en ce qu'il comprend en outre au moins une charge choisie parmi un agent opacifiant, un agent hydrofugeant ou encore un agent mouillant.

4. Produit filmogène selon l'une des revendications 1 à 3, caractérisé en ce que la substance filmogène est une hydroxypropylmethylcellulose.

5. Procédé pour la préparation d'un produit filmogène sous forme de particules granulées tel que défini à l'une des revendications 1 à 4, caractérisé en ce qu'il comprend le mouillage d'au moins une majeure partie, supérieure ou égale à environ 95 % en poids du poids total, de la substance filmogène sous forme de poudre, au moyen d'une solution de granulation liante, dans un mélangeur-granulateur ou dans un lit d'air fluidisé.

6. Procédé selon la revendication 5, caractérisé en ce que lorsque le mouillage précité est réalisé dans un mélangeur-granulateur, la masse humide obtenue est subséquemment broyée, puis séchée et éventuellement tamisée afin d'obtenir des particules granulées homogènes.

7. Procédé selon la revendication 5 ou 6, caractérisé en ce que la solution de granulation liante précitée comprend, dans un solvant, tel que de préférence l'eau :
- la totalité des pigments de coloration ;
- une faible partie, comprise entre environ 0,01 et environ 5 % en poids du poids total, de la substance filmogène ; et
- éventuellement l'agent plastifiant.

8. Procédé selon la revendication 7, caractérisé en ce que la solution de granulation liante précitée est obtenue par mélange d'une dispersion "mère" contenant une partie, comprise entre environ 10 et environ 50 % du poids total, du solvant ; les pigments de coloration et la faible partie précitée d'agent filmogène et d'une dispersion contenant la partie restante du solvant et éventuellement l'agent plastifiant.

9. Procédé selon la revendication 8, caractérisé en ce que préalablement au mélange précité des dispersions, la dispersion "mère" est broyée, de préférence dans un broyeur à billes, de façon à obtenir une granulométrie fine et homogène, par exemple inférieure à 150 µm.

10. Produits enrobés de préférence à usage agricole, alimentaire ou pharmaceutique, caractérisés en ce que l'enrobage a été réalisé à l'aide d'un produit filmogène tel que défini aux revendications 1 à 4, ou obtenu par la mise en oeuvre du procédé défini aux revendications 5 à 9.

## Claims

1. Film-forming product, free of α-cellulose, for coating solid forms, characterized in that it takes the form of homogeneous granular particles which can easily be dispersed in an aqueous or organic solvent and which make it possible to obtain a uniform non-matt film, in that its dry matter comprises :
- at least one ingestible, non-toxic film-forming substance in an amount of between 30 and 95% by weight;
- at least one colored pigment in an amount of between 5 and 50% by weight; and/or
- at least one edible plasticizer in an amount less than or equal to 25% by weight, and in that it is made from a process comprising wetting at least a major part, greater than or equal to about 95% by weight of the total weight, of the pulverulent film-forming substance with a binding granulating solution in a mixer-granulator or in a fluidized air bed.

2. Film-forming product according to claim 1, characterized in that its dry matter comprises:
- at least one ingestible, non-toxic film-forming substance in an amount of between 60 and 80% by weight, and preferably between 65 and 75% by weight;
- at least one colored pigment in an amount of 10 and 40% by weight, and preferably between 15 and 35% by weight; and/or
- at least one edible plasticizer in an amount less than or equal to 25% by weight, preferably between 5 and 15% and particularly preferably 10% by weight.

3. Film-forming product according to claim 1 or 2, characterized in that it also comprises at least one filler selected from an opacifier, a water repellent or else a wetting agent.

4. Film-forming product according to one of claims 1 to 3, characterized in that the film-forming substance is a hydroxypropyl methyl cellulose.

5. Process for the preparation of a film-forming product in the form of granular particles, such as defined in one of claims 1 to 4, characterized in that it comprises wetting at least a major part, greater than or equal to about 95% by weight of the total weight, of the pulverulent film-forming substance with a binding granulating solution in a mixer-granulator or in a fluidized air bed.

6. Process according to claim 5, characterized in that, if the above-mentioned wetting is carried out in a mixer-granulator, the moist mass obtained is subsequently ground and then dried and, if appropriate, sieved to give homogeneous granular particles.

7. Process according to claim 5 or 6, characterized in that the above-mentioned binding granulating solution comprises:
- all the colored pigments;
- a small part, of between about 0.01 and about 5% by weight of the total weight, of the film-forming substance; and
- if appropriate, the plasticizer,
in a solvent such as, preferably, water.

8. Process according to claim 7, characterized in that the above-mentioned binding granulating solution is obtained by mixing a "stock" dispersion containing a part, of between about 10 and about 50% of the total weight, of the solvent, the colored pigments and the above-mentioned small part of the film-forming agent with a dispersion containing the remainder of the solvent and, if appropriate, the plasticizer.

9. Process according to claim 8, characterized in that, prior to the above-mentioned mixing of the dispersions, the "stock" dispersion is ground, preferably in a ball mill, to give a fine and homogeneous particle size, for example below 150 µm.

10. Coated products, preferably for use in agriculture, foodstuffs or pharmacy, characterized in that they have been coated with a film-forming product such as defined in claims 1 to 4, or obtained by carrying out the process defined in claims 5 to 9.

## Patentansprüche

1. Filmbildendes, keine α-Cellulose enthaltendes Produkt, das für die Umhüllung fester Formen vorgesehen ist, dadurch gekennzeichnet, daß es in Form von homogenen, granulierten Partikeln vorliegt, die leicht in einem wäßrigen oder organischen Lösungsmittel dispergiert werden können und die Herstellung eines gleichmäßigen, nicht matten Films ermöglichen, daß seine Trockensubstanz
- mindestens eine nicht toxische, einnehmbare filmbildende Substanz in einem Mengenanteil von 30 bis 95 Gew.-%,
- mindestens ein färbendes Pigment in einem Mengenanteil von 5 bis 50 Gew.-% und/oder
- mindestens einen für Lebensmittel verwendbaren Weichmacher in einem Mengenanteil von 25 Gew.-% oder darunter
enthält und
daß es durch ein Verfahren herstellbar ist, das die Benetzung mindestens eines überwiegenden Teils der pulverförmigen filmbildenden Substanz, der größer als oder gleich etwa 95 Gew.-% des Gesamtgewicht ist, mit einer als Bindemittel dienenden Granulierlösung in einem Granuliermischer oder in einer Wirbelschicht umfaßt.

2. Filmbildendes Produkt nach Anspruch 1, dadurch gekennzeichnet, daß seine Trockensubstanz enthält:
- mindestens eine nicht toxische, einnehmbare filmbildende Substanz in einem Mengenanteil von 60 bis 80 Gew.-%, vorzugsweise 65 bis 75 Gew.-%,
- mindestens ein färbendes Pigment in einem Mengenanteil von 10 bis 40 Gew.-%, vorzugsweise 15 bis 35 Gew.-%, und/oder
- mindestens einen für Lebensmittel verwendbaren Weichmacher in einem Mengenanteil von 25 Gew.-% oder darunter, der vorzugsweise etwa 5 bis 15 Gew.-% und noch bevorzugter 10 Gew.-% beträgt.

3. Filmbildendes Produkt nach Anspruch 1 oder 2, dadurch gekennzeichnet, daß es außerdem mindestens einen Füllstoff enthält, der unter Trübungsmitteln, Hydrophobierungsmitteln und Netzmitteln ausgewählt ist.

4. Filmbildendes Produkt nach einem der Ansprüche 1 bis 3, dadurch gekennzeichnet, daß die filmbildende Substanz eine Hydroxypropylmethylcellulose ist.

5. Verfahren zur Herstellung eines filmbildenden Produkts in Form von granulierten Partikeln, das wie in einem der Ansprüche 1 bis 4 definiert ist, dadurch gekennzeichnet, daß es die Benetzung mindestens eines überwiegenden Teils der pulverförmigen filmbildenden Substanz, der größer als oder gleich etwa 95 Gew.-% des Gesamtgewicht ist, mit einer als Bindemittel dienenden Granulierlösung in einem Granuliermischer oder in einer Wirbelschicht umfaßt.

6. Verfahren nach Anspruch 5, dadurch gekennzeichnet, daß bei Durchführung der Benetzung in einem Granuliermischer die erhaltene feuchte Masse anschließend zerkleinert, dann getrocknet und schließlich gegebenenfalls gesiebt wird, um homogene granulierte Partikel zu erhalten.

7. Verfahren nach Anspruch 5 oder 6, dadurch gekennzeichnet, daß die als Bindemittel dienende Granulierlösung in einem Lösungsmittel, wie vorzugsweise Wasser,
- die Gesamtmenge an färbenden Pigmenten,
- einen kleinen Teil der filmbildenden Substanz, der etwa 0,01 bis etwa 5 Gew.-% des Gesamtgewichts beträgt, und
- gegebenenfalls den Weichmacher
enthält.

8. Verfahren nach Anspruch 7, dadurch gekennzeichnet, daß die als Bindemittel dienende Granulierlösung durch Mischen einer Grunddispersion, die einen Teil des Lösungsmittels, der etwa 10 bis etwa 50 % des Gesamtgewichts entspricht, die färbenden Pigmente und den oben erwähnten kleinen Teil des Filmbildners enthält, und einer Dispersion, die den verbleibenden Teil des Lösungsmittels und gegebenenfalls den Weichmacher enthält, hergestellt wird.

9. Verfahren nach Anspruch 8, dadurch gekennzeichnet, daß vor dem Vermischen der Dispersionen die Grunddispersion zerkleinert wird, vorzugsweise in einer Kugelmühle, um Partikel mit einer feinkörnigen und homogenen Partikelgröße beispielsweise unter 150 µm zu erhalten.

10. Umhüllte Erzeugnisse, vorzugsweise zur Verwendung auf dem Landwirtschafts-, Lebensmittel- oder Pharmaziesektor, dadurch gekennzeichnet, daß die Umhüllung mit einem filmbildenden Produkt, das wie in einem der Ansprüche 1 bis 4 definiert ist, oder durch Anwendung des in den Ansprüchen 5 bis 9 definierten Verfahrens hergestellt wurde, durchgeführt wurde.
